(19) Europäisches Patentamt
European Patent Office
Office européen des brevets

(11) Veröffentlichungsnummer: 0 406 993 A2

(12) # EUROPÄISCHE PATENTANMELDUNG

(21) Anmeldenummer: 90250169.1

(22) Anmeldetag: 03.07.90

(51) Int. Cl.[5]: C07D 498/06, C07D 519/00, A01N 43/90, //(C07D498/06, 265:00,221:00),(C07D498/06, 263:00,221:00),(C07D519/00, 498:00,471:00),(C07D519/00, 513:00,498:00)

(30) Priorität: 07.07.89 DE 3922847

(43) Veröffentlichungstag der Anmeldung: 09.01.91 Patentblatt 91/02

(84) Benannte Vertragsstaaten: AT BE CH DE DK ES FR GB GR IT LI LU NL SE

(71) Anmelder: SCHERING AKTIENGESELLSCHAFT
Berlin und Bergkamen
Müllerstrasse 170/178 Postfach 65 03 11
D-1000 Berlin 65(DE)

(72) Erfinder: Ganzer, Michael, Dr.
Eichborndamm 279
D-1000 Berlin 26(DE)
Erfinder: Dorfmeister, Gabriele, Dr.
Heiligenseestrasse 70
D-1000 Berlin 27(DE)
Erfinder: Franke, Wilfried, Dr.
Spiessergasse 6b
D-1000 Berlin 27(DE)
Erfinder: Johann, Gerhard, Dr.
Hermsdorfer Damm 147
D-1000 Berlin 28(DE)
Erfinder: Rees, Richard, Dr.
Speerweg 8
D-1000 Berlin 28(DE)

(54) **Substituierte Benzoxazolinon und Benzoxazinon-Derivate, Verfahren zu ihrer Herstellung und ihre Verwendung als Mittel mit herbizider Wirkung.**

(57) Die Erfindung betrifft neue substituierte Benzoxazolinon- und Benzoxazinonderivate der allgemeinen Formel I

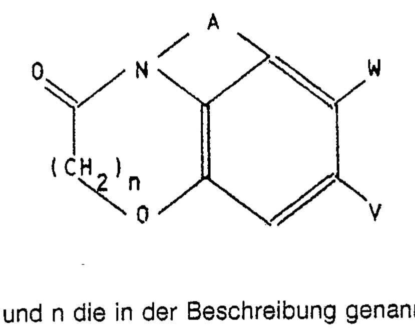

(I) ,

in der A, V, W und n die in der Beschreibung genannten Bedeutungen haben, Verfahren zu ihrer Herstellung und ihre Verwendung als Mittel mit herbizider Wirkung.

Die Erfindung betrifft auch neue Zwischenprodukte zur Herstellung von Verbindungen der allgemeinen Formel I, welche den allgemeinen Formel XXIII und XXIV

EP 0 406 993 A2

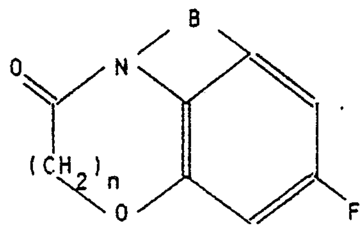

(XXIII)

und

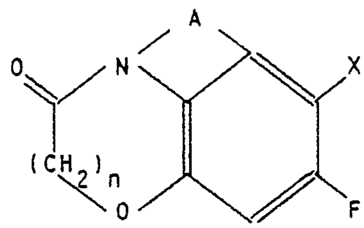

(XXIV) ,

in denen A, B, X und n die in der Beschreibung genannten Bedeutungen haben, entsprechen.

# SUBSTITUIERTE BENZOXAZOLINON- UND BENZOXAZINON-DERIVATE, VERFAHREN ZU IHRER HERSTELLUNG UND IHRE VERWENDUNG ALS MITTEL MIT HERBIZIDER WIRKUNG

Die Erfindung betrifft neue substituierte Benzoxazolinon- und Benzoxazinon-Derivate, Verfahren zu ihrer Herstellung und ihre Verwendung als Mittel mit herbizider Wirkung.

Es ist bereits bekannt, daß bestimmte Benzoxazinone (EP-A-0 170 191) und Benzofuranderivate (EP-A-0 271 170) herbizide Eigenschaften besitzen. Bei diesen bekannten Verbindungen ist jedoch die Herbizidwirkung nicht in allen Fällen ausreichend, oder es treten Selektivitätsprobleme in wichtiger landwirtschaftlichen Kulturen auf.

Aufgabe der vorliegenden Erfindung ist daher die Bereitstellung von neuen Verbindungen, die diese Nachteile nicht aufweisen und in ihren biologischen Eigenschaften den bisher bekannten überlegen sind.

Es wurde nun gefunden, daß substituierte Benzoxazolinon- und Benzoxazinon-Derivate der allgemeinen Formel I

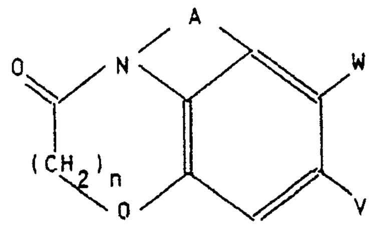

(I) ,

in der

A eine Gruppe $-CH_2-CH_2-CH_2-$, eine durch Methyl oder Ethyl subtituierte Gruppe $-CH_2-CH_2-CH_2-$, eine Gruppe $-CH=CH-CH_2-$, eine durch Methyl oder Ethyl substituierte Gruppe $-CH=CH-CH_2-$, eine Gruppe $-CH_2-CH=CH-$ oder eine durch Methyl oder Ethyl substituierte Gruppe $-CH_2-CH=CH-$,

n 0 oder 1,

V ein Wasserstoffatom, ein Fluoratom oder ein Chloratom,

W eine der Gruppen $W^1$ bis $W^4$ mit den allgemeinen Formeln

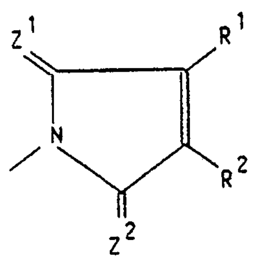

$W^1$ ,

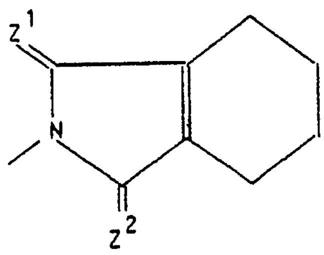

$W^2$ ,

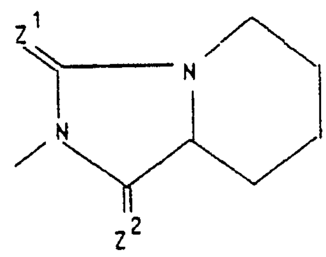

$W^3$

oder

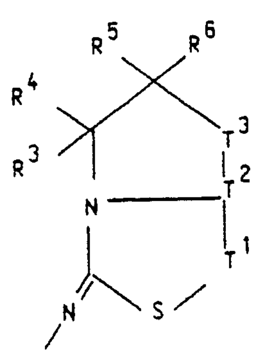

$W^4$ ,

$R^1$ ein Wasserstoffatom, einen $C_1$-$C_6$-Alkylrest oder einen ein- oder mehrfach durch Halogen substituierten

$C_1$-$C_6$-Alkylrest,
$R^2$ ein Wasserstoffatom, einen $C_1$-$C_6$-Alkylrest oder einen ein- oder mehrfach durch Halogen substituierten $C_1$-$C_6$-Alkylrest,
$Z^1$ ein Sauerstoffatom oder ein Schwefelatom,
$Z^2$ ein Sauerstoffatom oder ein Schwefelatom,
$T^1$-$T^2$ eine der Gruppen

$$-CH_2-CH- \quad \text{oder} \quad -N=C-$$

$T^3$ die Gruppe -D-E-,
D eine Gruppe $-(CR^7R^8)_o-$,
E ein Sauerstoffatom, ein Schwefelatom oder eine der Gruppen -SO-, $-SO_2-$, $-NR^9-$ oder $-CR^{10}R^{11}-$,
o 0 oder 1 und
$R^3$, $R^4$, $R^5$, $R^6$, $R^7$, $R^8$, $R^9$, $R^{10}$ und $R^{11}$ unabhängig voneinander ein Wasserstoffatom, einen $C_1$-$C_4$-Alkylrest oder einen Halogen-$C_1$-$C_4$-alkylrest
bedeuten, überraschenderweise eine hervorragende Verträglichkeit für Kulturpflanzen bei gleichzeitig interessanter herbizider Wirkung zeigen.

Der Ausdruck Halogen steht für Fluor, Chlor, Brom oder Jod. Die Bezeichnung Halogenalkyl besagt, daß ein oder mehrere Wasserstoffatome des Alkylrestes durch Halogen ersetzt sind.

Die Verbindungen der allgemeinen Formel I können gegebenenfalls in verschiedenen enantiomeren, diastereomeren oder geometrischen Formen anfallen und sind ebenfalls Gegenstand dieser Erfindung.

Die erfindungsgemäßen Verbindungen der allgemeinen Formel I lassen sich herstellen, indem man

A) falls W für $W^1$ oder $W^2$ und $Z^1$ und $Z^2$ für Sauerstoff stehen, eine Verbindung der allgemeinen Formel II

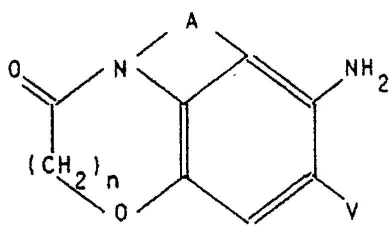

(II) ,

in der A, V und n die unter der allgemeinen Formel I genannten Bedeutungen haben, mit einer Verbindungen der allgemeinen Formeln III oder IV

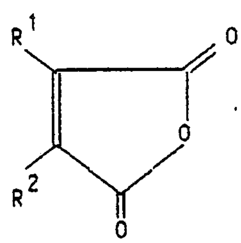

(III)

oder

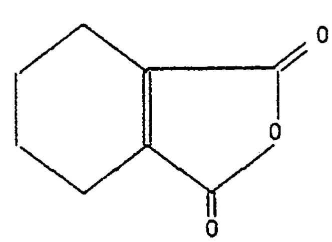

(IV) ,

in denen $R^1$ und $R^2$ die unter der allgemeinen Formel I genannten Bedeutungen haben, umsetzt,
B) falls W für $W^1$ oder $W^2$ und $Z^1$ und/oder $Z^2$ für Schwefel stehen, eine Verbindung der allgemeinen Formeln I a oder I b

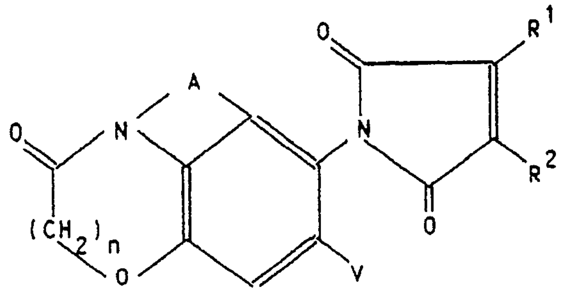

(I a)

oder

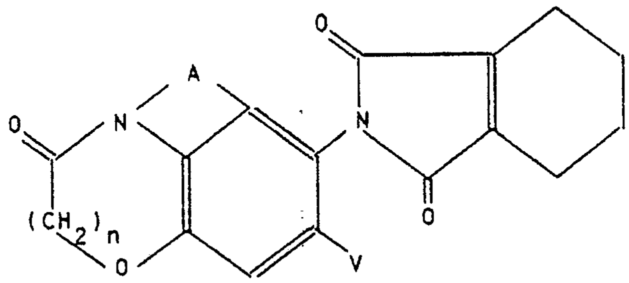

(I b) ,

in denen A, $R^1$, $R^2$, V und n die unter der allgemeinen Formel I genannten Bedeutungen haben, mit Phosphor(V)-sulfid oder Lawesson's Reagenz umsetzt,
C) falls W für $W^3$ steht, eine Verbindung der allgemeinen Formel V

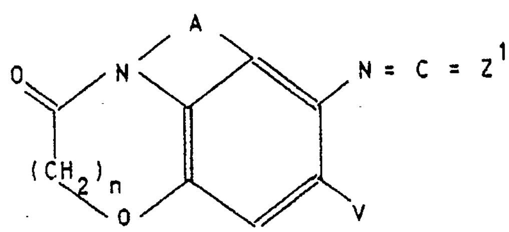

(V) ,

in der A, V, $Z^1$ und n die unter der allgemeinen Formel I genannten Bedeutungen haben, mit einer Verbindung der allgemeinen Formel VI

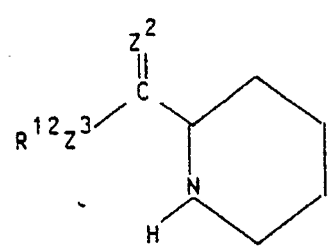

(VI) ,

in der $Z^2$ und $Z^3$ für ein Sauerstoffatom oder ein Schwefelatom und $R^{12}$ für ein Wasserstoffatom, ein Alkalimetallatom oder einen $C_1$-$C_4$-Alkylrest stehen, umsetzt,
D) falls W für $W^4$ und $T^1$-$T^2$ für die Gruppe

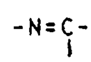

steht, eine Verbindung der allgemeinen Formel V a

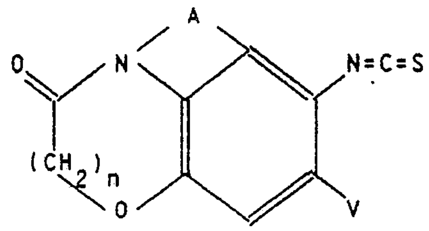

(V a),

in der A, V und n die unter der allgemeinen Formel 1 genannten Bedeutungen haben, mit einer Verbindung der allgemeinen Formel VII

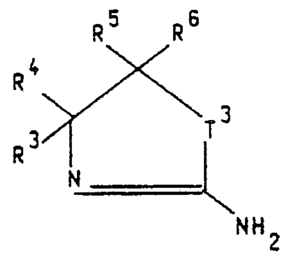

.(VII),

in der $R^3$, $R^4$, $R^5$, $R^6$ und $T^3$ die unter der allgemeinen Formel I genannten Bedeutungen haben, umsetzt und in Gegenwart eines Oxidationsmittels zum Thiadiazolring cyclisiert,

E) falls W für $W^4$ und $T^1$-$T^2$ für die Gruppe

$$-CH_2-CH-$$

steht, eine Verbindung der allgemeinen Formel VIII

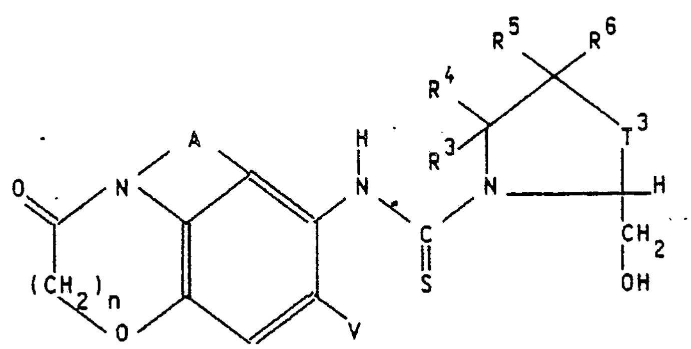

(VIII),

in der A, $R^3$, $R^4$, $R^5$, $R^6$, $T^3$, V und n die unter der allgemeinen Formel I genannten Bedeutungen haben, unter sauren Bedingungen cyclisiert.

Die Umsetzung gemäß der Verfahrensvarianten A) wird zweckmäßig bei 20°C bis 200°C gegebenenfalls in einem geeigneten Lösungsmittel durchgeführt, wobei das Anhydrid in einem Molverhältnis von 1 bis 3 Äquivalenten zu 1 Äquivalent Anilin der Formel II eingesetzt wird. Die Reaktionszeit beträgt 1 bis 24 Stunden.

Es ist zweckmäßig, die Reaktion in Gegenwart einer Säure, wie beispielsweise Essigsäure, durchzuführen, zum Beispiel indem man in Essigsäure als Lösungsmittel arbeitet. Es ist aber auch möglich, die beiden Reaktionspartner unter Verwendung eines inerten Lösungsmittels, wie zum Beispiel Dichlormethan oder Dimethylsulfoxid, zur Reaktion zu bringen und die intermediär entstandenen Additionsprodukte der allgemeinen Formeln IX beziehungsweise X

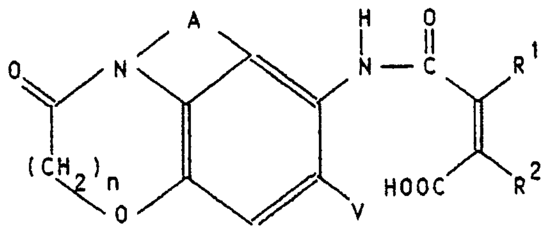

(IX)

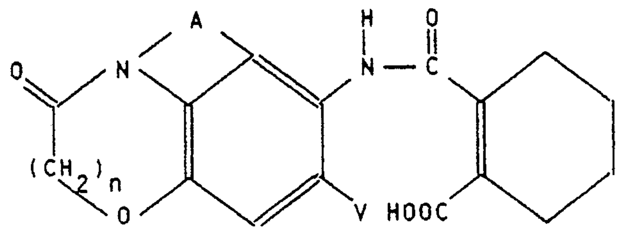

(X) ,

in denen A, $R^1$, $R^2$, V und n die unter der allgemeinen Formel I genannten Bedeutungen haben, mit Säureanhydriden, wie zum Beispiel Acetanhydrid, zu cyclisieren.

Die als Ausgangsmaterial verwendeten Aniline der allgemeinen Formel II sind zum Teil bekannt (DE-OS 32 34 529) oder lassen sich nach analogen Verfahren herstellen, zum Beispiel nach folgendem Schema:

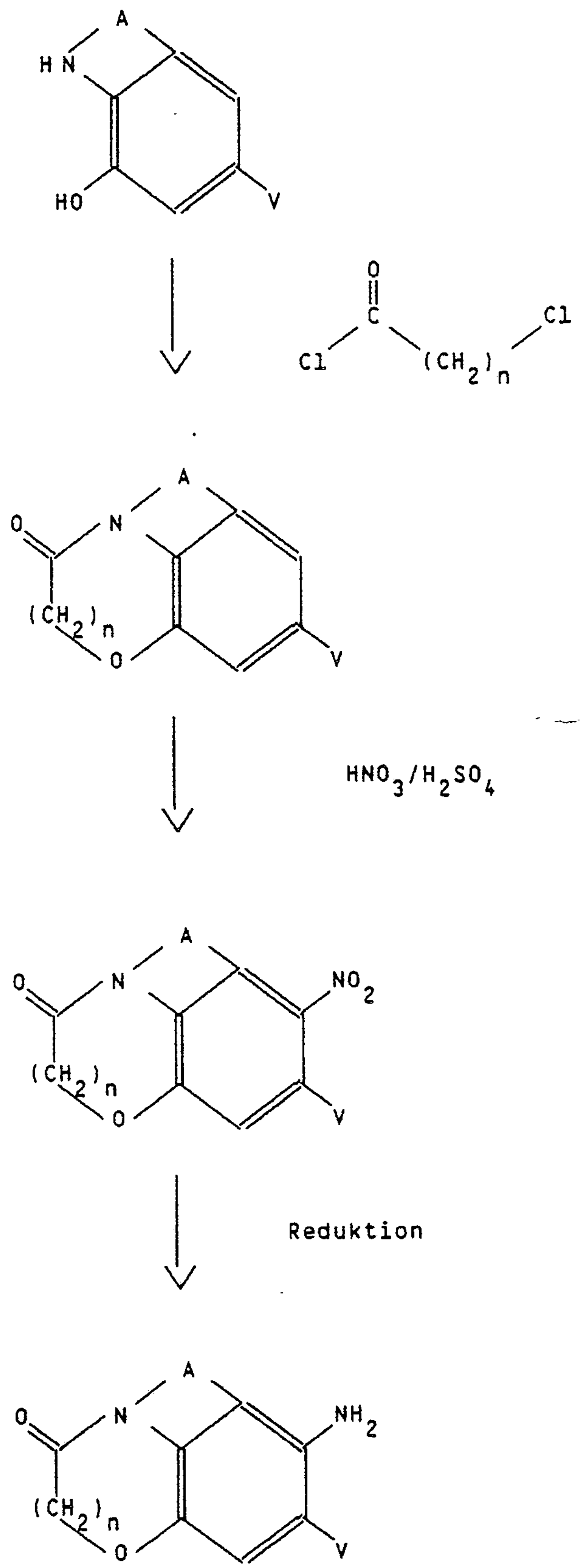


(XI)

(XII)

(XIII)

(II)

Eine Verbindung der allgemeinen Formel XI, in der A und V die unter der allgemeinen Formel I genannten Bedeutungen haben, wird mit Phosgen (n = 0) oder Chloracetylchlorid (n = 1) umgesetzt. Die Umsetzung wird zweckmäßig bei -20°C bis +150°C in einem geeigneten Lösungsmittel, wie zum Beispiel Hexan, Toluol, Aceton, Methylisobutylketon, Methylenchlorid oder Diethylether, gegebenenfalls unter Zugabe einer geeigneten organischen oder anorganischen Base und gegebenenfalls im Zweiphasensystem mit

Wasser durchgeführt. Die Reaktionszeit beträgt 0,5 bis 24 Stunden. Die substituierten Benzoxazinone beziehungsweise Benzoxazolinone der allgemeinen Formel XII, in denen A, V und n die unter der allgemeinen Formel I genannten Bedeutungen haben, werden mit Salpetersäure oder einem anorganischen Salz der Salpetersäure, gegebenenfalls unter Zugabe einer weiteren anorganischen Säure, wie zum Beispiel Schwefelsäure, nitriert. Die Umsetzung wird zweckmäßig bei -50°C bis +50°C in einem geeigneten Lösungsmittel oder ohne Lösungsmittel durchgeführt. Die Reaktionszeit beträgt 0,5 bis 24 Stunden. Die Nitrobenzoxazinon- beziehungsweise Nitrobenzoxazolinonderivate der allgemeinen Formel XIII, in der A, V und n die unter der allgemeinen Formel I genannten Bedeutungen haben, werden mit Wasserstoff in Gegenwart eines Katalysators und in Gegenwart eines Verdünnungsmittels gegebenenfalls unter Druck zu den entsprechenden Aminoverbindungen der allgemeinen Formel II reduziert.

Es können aber auch andere Methoden zur Reduktion von aromatischen Nitroverbindungen verwendet werden, wie sie beispielsweise im Houben-Weyl, Methoden der organischen Chemie, Band 11,1 Seite 360 ff., Reduktion von Nitroverbindungen, beschrieben sind.

Es ist aber auch möglich, die Aniline der allgemeinen Formel II nach folgendem Schema aufzubauen:

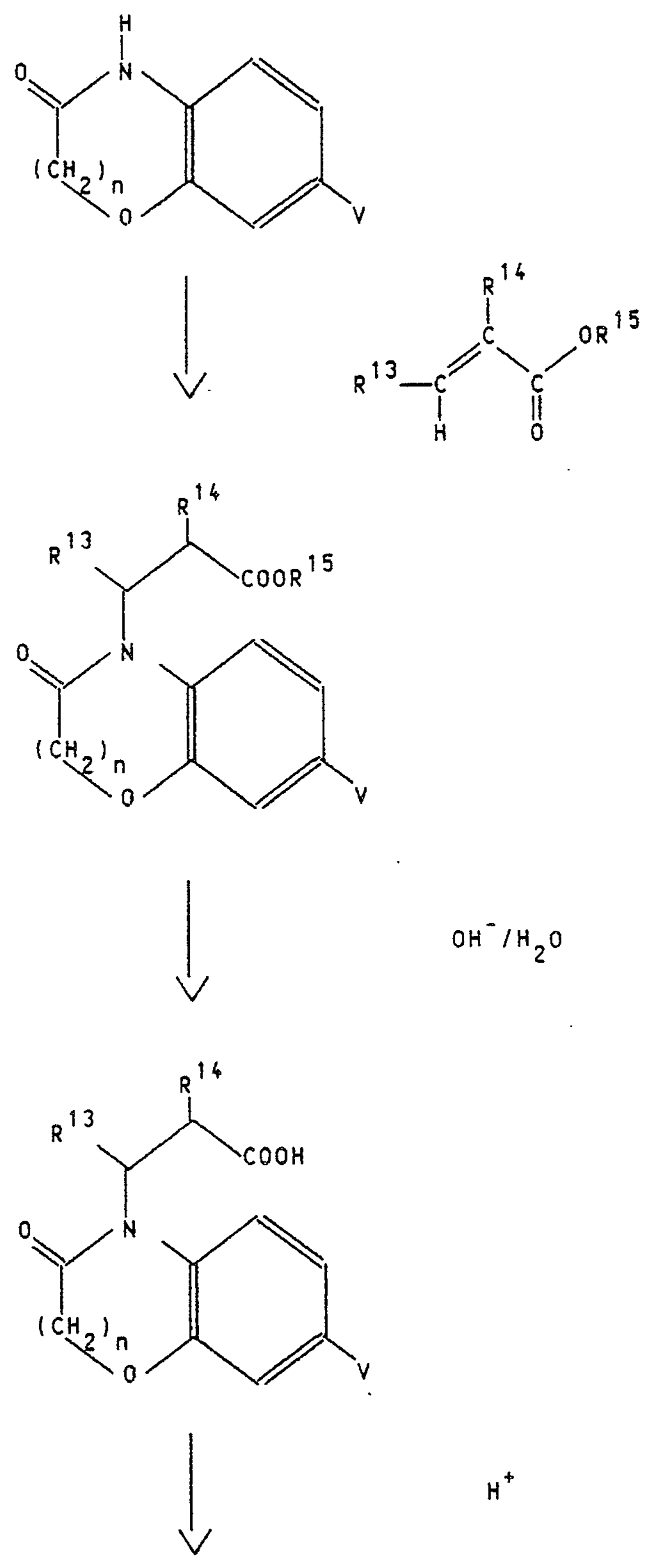


(XIV)

(XV)

(XVI)

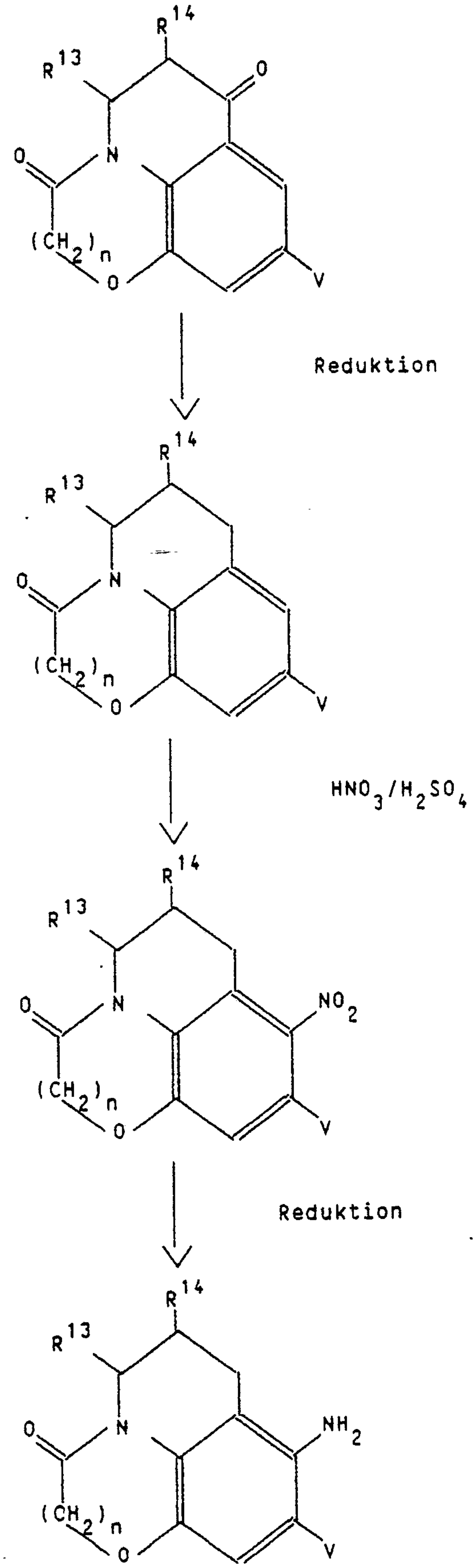


(XVII)

(XVIII)

(XIX)

(XX)

Ein Benzoxazolinon- oder Benzoxazinon-Derivat der allgemeinen Formel XIV, in der V und n die unter der allgemeinen Formel I genannten Bedeutungen haben, wird mit einem Acrylsäureester oder β-Brompropionsäureester in einem inerten Lösungsmittel in Gegenwart katalytischer bis äquivalenter Mengen einer organischen oder anorganischen Base bei einer Temperatur zwischen -50°C und +150°C alkyliert. Die Reaktionszeit beträgt 0,5 bis 20 Stunden.

Die Ester der allgemeinen Formel XV, in denen V und n die unter der allgemeinen Formel I genannten Bedeutungen haben, $R^{13}$ und $R^{14}$ unabhängig voneinander für Wasserstoff, Methyl oder Ethyl stehen und $R^{15}$ Methyl oder Ethyl bedeutet, können nach den üblichen Verfahren entweder im sauren oder im alkalischen Milien zu den entsprechenden Carbonsäuren der allgemeinen Formel XVI verseift werden.

Die anschließende Cyclisierung zu Verbindungen der allgemeinen Formel XVII erfolgt mit oder ohne Lösungsmittel gegebenenfalls unter Zugabe eines wasserentziehenden Mittels, beispielsweise indem man die Carbonsäuren in Polyphosphorsäure oder Schwefelsäure erwärmt.

Es kann aber auch beispielsweise in Toluol in Gegenwart katalytischer bis äquivalenter Mengen anorganischer oder organischer Säuren, wie beispielsweise p-Toluolsulfonsäure, am Wasserabschneider gearbeitet werden.

Die Reduktion zu einer Verbindung der allgemeinen Formel XVIII kann unter den üblichen Bedingungen der Reduktion einer Carbonylgruppe, wie sie beispielsweise in "Advanced Organic Chemistry" Jerry March, Mc Graw-Hill International Bock Company 1977, Seite 1119 unter "Reaktions involving Replacement of Oxygen by Hydrogen' beschrieben sind, durchgeführt werden, zum Beispiel indem mit Zink/Quecksilber in wäßrigen anorganischen Säuren, wie beispielsweise Salzsäure, gearbeitet wird. Andererseits kann auch in wäßrig alkalischen Milieu mit Hydrazinhydrat in wäßriger Natronlauge gearbeitet werden. Die Reduktion kann aber auch beispielsweise mit Silanen in organischen Lösungsmitteln gegebenenfalls in Gegenwart von organischen Säuren, wie zum Beispiel Trifluoressigsäure, erfolgen.

Die Benzoxazolinon- beziehungsweise Benzoxazinon-Derivate der allgemeinen Formel XVIII, in der V und n die unter der allgemeinen Formel I genannte Bedeutung haben und $R^{13}$ und $R^{14}$ Wasserstoff, Methyl oder Ethyl bedeuten, werden mit Salpetersäure oder einem anorganischen oder organischen Salz der Salpetersäure, gegebenenfalls unter Zugabe einer weiteren anorganischen Säure, wie zum Beispiel Schwefelsäure, nitriert.

Die Umsetzung wird zweckmäßig bei -50°C bis +50°C in einem geeigneten Lösungsmittel oder ohne Lösungsmittel durchgeführt. Die Reaktionszeit beträgt 0,5 bis 24 Stunden.

Die Nitrobenzoxazolinon- beziehungsweise Nitrobenzoxazinon-Derivate der allgemeinen Formel XIX werden mit Wasserstoff in Gegenwart eines Katalysators und in Gegenwart eines Verdünnungsmittels gegebenenfalls unter Druck zu den entsprechenden Aminoverbindungen der allgemeinen Formel XX reduziert.

Die Umwandlung der Carbonylgruppe in Position 7 der Verbindungen der allgemeinen Formel XVII in eine Doppelbindung in Position 6, 7 der Verbindungen der allgemeinen Formel XXII kann auf verschiedenen Wegen durchgeführt werden, zum Beispiel nach folgendem Schema:

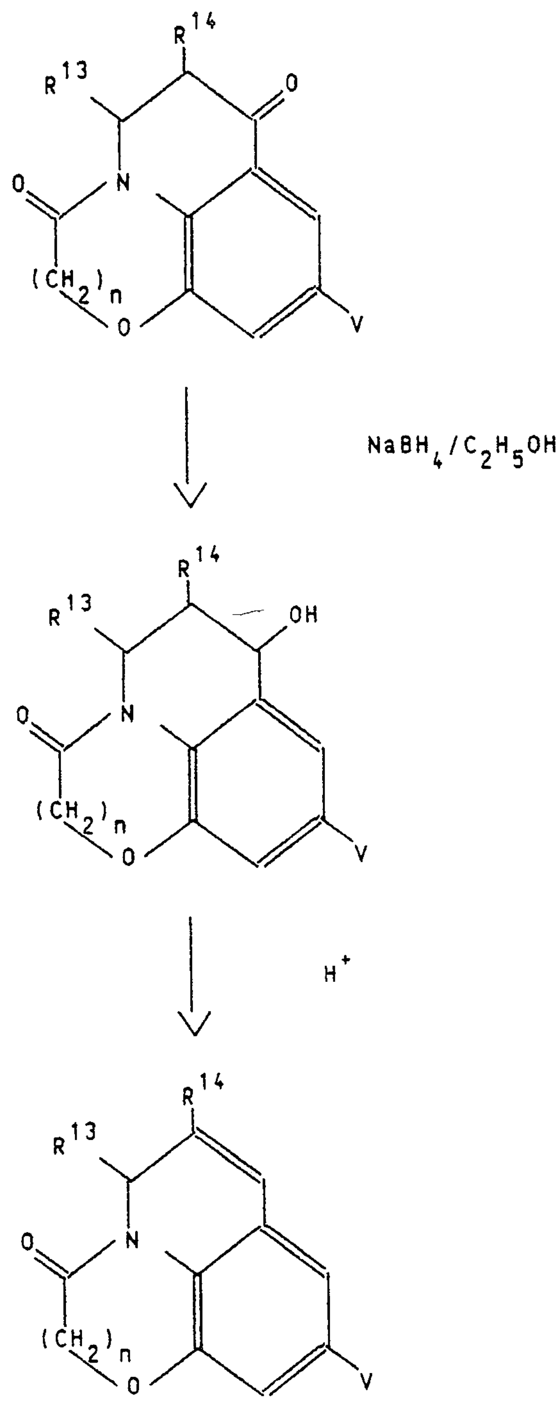


(XVII)

(XXI)

(XXII)

Man kann beispielsweise die Carbonylgruppe in Position 7 der Verbindungen der allgemeinen Formel XVII auf dem allgemein bekannten Weg zu einer Hydroxygruppe, wie in den Verbindungen der allgemeinen Formel XXI, reduzieren und die Hydroxygruppe unter den üblichen Bedingungen eliminieren.

Als Beispiel für Reduktionsmittel seien komplexe Metallhydride, wie zum Beispiel Natriumborhydrid, in alkoholischer Lösung, zum Beispiel in Ethanol, genannt.

Die Eliminierung der Hydroxygruppe kann in einem inerten Lösungsmittel gegebenenfalls in Gegenwart von katalytischen bis äquivalenten Mengen organischer oder anorganischer Säuren gegebenenfalls unter Zugabe von wasserentziehenden Mitteln durchgeführt werden. Es kann aber auch das entstehende Wasser azeotrop abdestilliert werden.

Als Säuren kommen Salzsäure, Schwefelsäure oder p-Toluolsulfonsäure in Frage, als Lösungsmittel können beispielsweise aromatische Kohlenwasserstoffe, wie Benzol, Toluol oder Xylol, verwendet werden. Die Reaktionszeit beträgt 0,25 bis 24 Stunden. Die Temperaturen können zwischen Raumtemperatur und

200°C liegen, bevorzugt wird die Siedetemperatur der Lösungsmittel.

Man kann aber auch Verbindungen der allgemeinen Formel XVII mit p-Toluolsulfonsäurehydrazid umsetzen, und das gebildete Hydrazon mit einer organischen oder anorganischen Base unter Stickstoffentwicklung eliminieren. Als Beispiel einer organischen Base seien Lithiumdiisopropylamid oder n-Butyllithium als anorganische Basen Natriumhydrid genannt.

Die so entstehenden Verbindungen der allgemeinen Formel XXII werden dann nitriert und reduziert, wie es für die Verbindungen der allgemeinen Formel XVIII und XIX weiter oben beschrieben wurde.

Ein Teil der bei der Herstellung der Aniline der allgemeinen Formel II anfallenden Zwischenprodukte und auch ein Teil oder Aniline sind neu und ebenfalls Gegenstand dieser Erfindung.

Es handelt sich hierbei um Verbindungen der allgemeinen Formel XXIII

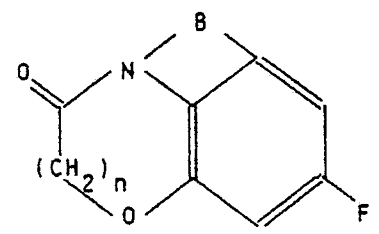

(XXIII) ,

in der B eine durch Methyl oder Ethyl subtituierte Gruppe $-CH_2-CH_2-CH_2-$, eine Gruppe $-CH=CH-CH_2-$, eine durch Methyl oder Ethyl substituierte Gruppe $-CH=CH-CH_2-$, eine Gruppe $-CH_2-CH=CH-$, eine durch Methyl oder Ethyl substituierte Gruppe $-CH_2-CH=CH-$, eine Gruppe $-CH_2-CH_2-CHOH-$, eine durch Methyl oder Ethyl substituierte Gruppe $-CH_2-CH_2-CHOH-$, eine Gruppe $-CH_2-CH_2-CO-$oder eine durch Methyl oder Ethyl substituierte Gruppe $CH_2-CH_2-CO-$, und
n 0 oder 1
bedeuten, und Verbindungen der allgemeinen Formel XXIV

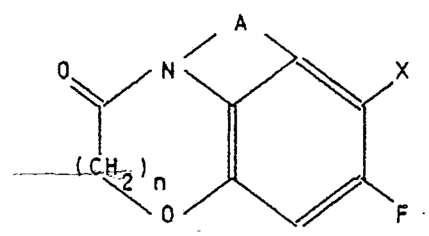

(XXIV) ,

in der
A eine Gruppe $-CH_2-CH_2-CH_2-$, eine durch Methyl oder Ethyl subtituierte Gruppe $-CH_2-CH_2-CH_2-$, eine Gruppe $-CH=CH-CH_2-$, eine durch Methyl oder Ethyl substituierte Gruppe $-CH=CH-CH_2-$, eine Gruppe $-CH_2-CH=CH-$ oder eine durch Methyl oder Ethyl substituierte Gruppe $-CH_2-CH=CH-$,
X eine Nitrogruppe oder eine Aminogruppe und
n 0 oder 1,
bedeuten.

Die Umsetzung gemäß Verfahrensvariante B) wird zweckmäßig bei 0 bis 150°C in einem geeigneten Lösungsmittel durchgeführt. Als Lösungsmittel kommen Ether, wie zum Beispiel Tetrahydrofuran, aromatische Kohlenwasserstoffe, wie zum Beispiel Toluol, Heteroaromaten, wie zum Beispiel Pyridin, und andere gegenüber den Reaktionspartnern inerte Lösungsmittel in Frage. Gegebenenfalls können der Reaktionslösung auch Basen, wie zum Beispiel Triethylamin, zugesetzt werden. Die Reaktionszeit beträgt 1 bis 24 Stunden.

Die Umsetzung gemäß der Verfahrensvariante C) wird zweckmäßigerweise so durchgeführt, daß man die Ausgangsverbindungen in einem organischen Lösungsmittel gegebenenfalls unter Zusatz katalytischer bis äquimolarer Mengen eines organischen Amins, wie zum Beispiel Triethylamin oder Pyridin, über längere Zeit, wie zum Beispiel 0,5 bis 15 Stunden, bei einer Temperatur von 20°C bis zur Siedetemperatur des jeweiligen Lösungsmittels umsetzt.

Als Lösungsmittel eignen sich alle unter den Reaktionsbedingungen inerten organischen Lösungsmittel, wie zum Beispiel Toluol, Aceton, Acetonitril, Dimethylformamid, Ether, Tetrahydrofuran, Dioxan, Ethanol und

Methanol, oder auch Wasser.

Gegebenenfalls können nicht cyclisierte Zwischenprodukte der allgemeinen Formel XXV

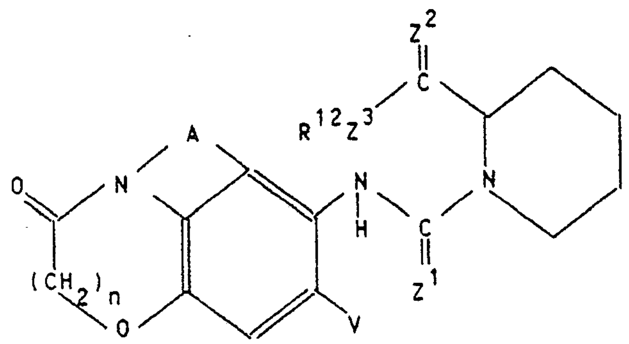

(XXV) ,

in denen A, V, $Z^1$, $Z^2$ und n die unter der allgemeinen Formel genannten Bedeutungen haben, $Z^3$ für ein Sauerstoffatom oder ein Schwefelatom und $R^{12}$ für ein Wasserstoffatom, ein Alkalimetallatom oder einen $C_1$-$C_4$-Alkylrest stehen, durch Zugabe einer Mineralsäure, wie zum Beispiel Salzsäure, oder Zugabe einer Base, wie zum Beispiel Natriummethanolat, und gegebenenfalls weiteres Erhitzen bis zur Siedetemperatur des Lösungsmittels cyclisiert werden.

Die für die Umsetzung benötigten Isocyanate und Isothiocyanate der allgemeinen Formel V lassen sich nach den üblichen Verfahren aus den Anilinen der allgemeinen Formel II herstellen.

Die Umsetzung gemäß Verfahrensvariante D) erfolgt in einem inerten Lösungsmittel, wie zum Beispiel Ether, Methylenchlorid, Chloroform oder Ethylacetat, bei einer Temperatur von -50°C bis +50°C durch Umsetzung einer Verbindung der allgemeinen Formel V a, in der A, V und n die unter der allgemeinen Formel I genannten Bedeutungen haben, mit einer Verbindung der allgemeinen Formel VII, in der $R^3$, $R^4$, $R^5$, $R^6$ und $T^3$ die unter der allgemeinen Formel I genannten Bedeutungen haben. Die Reaktionszeit beträgt 0,5 bis 10 Stunden. Die erhaltene Verbindung der allgemeinen Formel XXVI

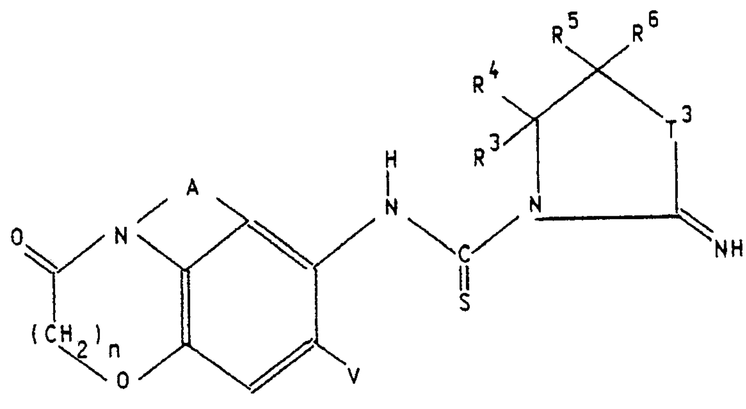

(XXVI)

ist thermisch instabil und wird deshalb vorzugsweise ohne Isolierung in die nächste Reaktion eingesetzt.

Die Ringbildung wird unter Verwendung eines Oxidationsmittels in einem organischen Lösungsmittel durchgeführt. Als organisches Lösungsmittel kommt ein inertes Lösungsmittel, wie Methylenchlorid, Chloroform, N,N-Dimethylformamid und Ethylacetat, in Frage. Die Kondensationsreaktion unter Ringbildung kann in Gegenwart von wirksamen Säureakzeptoren, je nach Art des Oxidationsmittels, durchgeführt werden. Als Säureakzeptoren kommen organische Basen, wie Triethylamin, Pyridin, Dimethylanilin, anorganische Basen, wie Natriumhydroxid und Natriumcarbonat, in Frage. Als Oxidationsmittel werden Brom, Chlor, Natriumhypochlorit oder andere eingesetzt.

Die Umsetzung gemäß Verfahrensvariante E) wird zweckmäßigerweise so durchgeführt, daß man Thioharnstoffe der allgemeinen Formel VIII mit einer starken Mineralsäure, wie zum Beispiel Salzsäure, Bromwasserstoffsäure oder auch Schwefelsäure, in einem Temperaturbereich von 0°C bis 150°C cyclisiert. Die Reaktionszeit beträgt 0,5 bis 24 Stunden. Die Cyclisierung kann auch durch Umsetzung mit Thionylchlorid erfolgen.

Gegebenenfalls kann auch unter Zugabe von organischen Lösungsmitteln, wie zum Beispiel Diethylether, Tetrahydrofuran, Dioxan, Methanol oder Ethanol, gearbeitet werden. Nach dem Neutralisieren mit zum Beispiel Natronlauge wird wie üblich aufgearbeitet. Die Salze der Verbindungen werden erhalten, wenn man vor dem Neutralisieren nach üblichen Verfahren aufarbeitet.

Die bei den einzelnen Verfahrensvarianten genannten Ausgangsmaterialien sind, sofern die Herstellung nicht beschrieben ist, bekannt oder lassen sich analog zu an sich bekannten Verfahren herstellen.

Die Aufarbeitung der nach Verfahrensvarianten A) bis E) hergestellten erfindungsgemäßen Verbindungen erfolgt in der üblichen Art und Weise. Eine Aufreinigung erfolgt durch Kristallisation oder Säulenchromatographie.

Die erfindungsgemäßen Verbindungen stellen in der Regel farblose bis schwach gelb gefärbte kristalline oder zähflüssige Substanzen dar, die zum Teil gut löslich in Ethern, wie zum Beispiel Tetrahydrofuran, Alkoholen, wie zum Beispiel Methanol oder Ethanol, halogenierten Kohlenwasserstoffen, wie zum Beispiel Methylenchlorid oder Chloroform, Sulfoxiden, wie zum Beispiel Dimethylsulfoxid, oder Estern, wie zum Beispiel Essigester, sind.

Die erfindungsgemäßen Verbindungen zeigen eine gute herbizide Wirkung bei breitblättrigen Unkräutern und Gräsern. Ein selektiver Einsatz der erfindungsgemäßen Wirkstoffe ist in verschiedenen Kulturen möglich, zum Beispiel in Raps, Rüben, Sojabohnen, Baumwolle, Reis, Gerste, Weizen und anderen Getreidearten. Dabei sind einzelne Wirkstoffe als Selektivherbizide in Rüben, Baumwolle, Soja und Getreide besonders geeignet. Ebenso können die Verbindungen zur Unkrautbekämpfung in Dauerkulturen, wie zum Beispiel Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, und zur selektiven Unkrautbekämpfung in einjährigen Kulturen eingesetzt werden.

Die erfindungsgemäßen Verbindungen können zum Beispiel bei den folgenden Pflanzengattungen verwendet werden:

Dikotyle Unkräuter der Gattungen Sinapis, Lepidium, Galium, Stellaria, Matricaria, Anthemis, Galinsoga, Chenopodium, Brassica, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Lamium, Veronica, Abutilon, Datura, Viola, Galeopsis, Papaver, Centaurea und Chrysanthemum.

Monokotyle Unkräuter der Gattungen Avena, Alopecurus, Echinochloa, Setaria, Panicum, Digitaria, Poa, Eleusine, Brachiaria, Lolium, Bromus, Cyperus, Agropyron, Sagittaria, Monochoria, Fimbristylis, Eleocharis, Ischaemum und Apera.

Die Aufwandmengen schwanken je nach Anwendungsart im Vor- und Nachauflauf in Grenzen zwischen 0,001 bis 5 kg/ha.

Die erfindungsgemäßen Wirkstoffe können auch als Defoliant, Desiccant und als Krautabtötungsmittel verwendet werden.

Die erfindungsgemäßen Verbindungen können entweder allein, in Mischung miteinander oder mit anderen Wirkstoffen angewendet werden. Gegebenenfalls können andere Pflanzenschutz- oder Schädlingsbekämpfungsmittel je nach dem gewünschten Zweck zugesetzt werden. Sofern eine Verbreiterung des Wirkungsspektrums beabsichtigt ist, können auch andere Herbizide zugesetzt werden.

Beispielsweise eignen sich als herbizid wirksame Mischungspartner diejenigen Wirkstoffe, die in Weed Abstracts, Vol. 38, No. 3, 1989, unter dem Titel "List of common names and abbreviations employed for currently used herbicides and plant growth regulators in Weed Abstracts" aufgeführt sind.

Eine Förderung der Wirkintensität und der Wirkungsgeschwindigkeit kann zum Beispiel durch wirkungssteigernde Zusätze, wie organische Lösungsmittel, Netzmittel und Öle, erzielt werden. Solche Zusätze lassen daher gegebenenfalls eine Verringerung der Wirkstoffdosierung zu.

Zweckmäßig werden die gekennzeichneten Wirkstoffe oder deren Mischungen in Form von Zubereitungen, wie Pulvern, Streumitteln, Granulaten, Lösungen, Emulsionen oder Suspensionen, unter Zusatz von flüssigen und/oder festen Trägerstoffen beziehungsweise Verdünnungsmitteln und gegebenenfalls Haft-, Netz-, Emulgier- und/oder Dispergierhilfsmitteln angewandt.

Geeignete flüssige Trägerstoffe sind zum Beispiel aliphatische und aromatische Kohlenwasserstoffe, wie Benzol, Toluol, Xylol, Cyclohexanon, Isopho ron, Dimethylsulfoxid, Dimethylformamid, weiterhin Mineralölfraktionen und Pflanzenöle.

Als feste Trägerstoffe eignen sich Mineralien, zum Beispiel Bentonit, Silicagel, Talkum, Kaolin, Attapulgit, Kalkstein, und pflanzliche Produkte, zum Beispiel Mehle.

An oberflächenaktiven Stoffen sind zu nennen zum Beispiel Calciumligninsulfonat, Polyethylenalkylphenylether, Naphthalinsulfonsäuren und deren Salze, Phenolsulfonsäuren und deren Salze, Formaldehydkondensate, Fettalkoholsulfate sowie substituierte Benzolsulfonsäuren und deren Salze.

Der Anteil des beziehungsweise der Wirkstoffe(s) in den verschiedenen Zubereitungen kann in weiten Grenzen variieren. Beispielsweise enthalten die Mittel etwa 10 bis 90 Gewichtsprozent Wirkstoff, etwa 90 bis

10 Gewichtsprozent flüssige oder feste Trägerstoffe sowie gegebenenfalls bis zu 20 Gewichtsprozent oberflächenaktive Stoffe.

Die Ausbringung der Mittel kann in üblicher Weise erfolgen, zum Beispiel mit Wasser als Träger in Spritzbrühmengen etwa 100 bis 1000 Liter/ha. Eine Anwendung der Mittel im sogenannten Low-Volume und Ultra-Low-Volume-Verfahren ist ebenso möglich wie ihre Applikation in Form von sogenannten Mikrogranulaten.

Die Herstellung dieser Zubereitungen kann in an sich bekannter Art und Weise, zum Beispiel durch Mahl- oder Mischverfahren, durchgeführt werden. Gewünschtenfalls können Zubereitungen der Einzelkomponenten auch erst kurz vor ihrer Verwendung gemischt werden, wie es zum Beispiel im sogenannten Tankmixverfahren in der Praxis durchgeführt wird.

Zur Herstellung der verschiedenen Zubereitungen werden zum Beispiel die folgenden Bestandteile eingesetzt:

A) **Spritzpulver**

1.) 20 Gewichtsprozent Wirkstoff
68 Gewichtsprozent Kaolin
10 Gewichtsprozent Calciumsalz der Ligninsulfonsäure
2 Gewichtsprozent Dialkylnaphthalinsulfonat

2.) 40 Gewichtsprozent Wirkstoff
25 Gewichtsprozent Kaolin
25 Gewichtsprozent kolloidale Kieselsäure
8 Gewichtsprozent Calciumsalz der Ligninsulfonsäure
2 Gewichtsprozent Natriumsalz des N-Methyl-N-oleyl-taurins

B) **Paste**

45 Gewichtsprozent Wirkstoff
5 Gewichtsprozent Natriumaluminiumsilikat
15 Gewichtsprozent Cetylpolyglycolether mit 8 Mol Ethylenoxid
2 Gewichtsprozent Spindelöl
10 Gewichtsprozent Polyethylenglycol
23 Gewichtsprozent Wasser

C) **Emulsionskonzentrat**

20 Gewichtsprozent Wirkstoff
75 Gewichtsprozent Isophoron
2 Gewichtsprozent ethoxyliertes Rizinusöl
3 Gewichtsprozent Calciumsalz der Dodecylphenylsulfonsäure

Die folgenden Beispiele erläutern die Herstellung der erfindungsgemäßen Verbindungen:

**Beispiel 1.01** (Verfahren A)

N-(3-Oxo-3,5,6,7-tetrahydro-2H-pyrido[1,2,3-de]-1,4-benzoxazin-8-yl)-3,4,5,6-tetrahydrophthalimid

Eine Lösung von 1,4 g 8-Amino-3,5,6,7-tetrahydro-2H-pyrido[1,2,3-de]-1,4-benzoxazin-3-on und 1,25 g 3,4,5,6-Tetrahydrophthalsäureanhydrid in 20 ml Eisessig wird 2 Stunden unter Rückfluß erhitzt. Nach dem Erkalten wird die Reaktionslösung in 150 ml Eiswasser gegossen, und die ausgefallenen Kristalle werden abgesaugt. Man trocknet bei 50°C im Vakuum und kristallisiert aus Hexan/Essigester um.

Ausbeute: 2,1 g = 90 % der Theorie
Fp.: 219 - 222 °C

In analoger Weise wurden auch die folgenden Verbindung nach Verfahren A hergestellt:

| Beispiel Nr. | Name | Physikalische Konstante |
|---|---|---|
| 1.02 | N-(9-Fluor-3-oxo-3,5,6,7-tetrahydro-2H-pyrido[1,2,3-de]-1,4-benzoxazin-8-yl)-3,4,5,6-tetrahydrophthalimid | Fp.: 220°C |
| 1.03 | N-(9-Fluor-3-oxo-3,5,6,7-tetrahydro-2H-pyrido[1,2,3-de]-1,4-benzoxazin-8-yl)-3,4-dimethyl-3-pyrrolin-2,5(1H)-dion | Fp.: 204°C |
| 1.04 | N-(9-Fluor-3-oxo-3,5-dihydro-2H-pyrido-[1,2,3-de]-1,4-benzoxazin-8-yl)-3,4,5,6-tetrahydrophthalimid | Fp.: 216-218°C |
| 1.05 | N-(9-Fluor-5-methyl-3-oxo-3,5,6,7-tetrahydro-2H-pyrido[1,2,3-de]-1,4-benzoxazin-8-yl)-3,4,5,6-tetrahydrophthalimid | Fp.: 92-94 °C |

**Beispiel 2.01** (Verfahren C)

2-(3-Oxo3,5,6,7-tetrahydro-2H-pyrido[1,2,3-de]-1,4-benzoxazin-8-yl)-perhydroimidazo[1,5-a]pyridin-1,3-dion

Man löst 3,2 g 3-Oxo-3,5,6,7-tetrahydro-2H-pyrido[1,2,3-de]-1,4-benzoxazin-8-yl-isocyanat in 25 ml Tetrahydrofuran und gibt 2,1 ml Piperidin-2-carbonsäureethylester hinzu. Es wird 10 Stunden unter Rückfluß gekocht. Nach dem Abkühlen wird das Lösungsmittel im Vakuum abgezogen und der Rückstand über eine Kieselgelsäule mit Essigester/Hexan-Gemischen getrennt.
Ausbeute: 2,4 g = 50 % der Theorie
Fp.: 206°C

In analoger Weise wurde auch die folgende Verbindung nach Verfahren C hergestellt:

| Beispiel Nr. | Name | Physikalische Konstante |
|---|---|---|
| 2.02 | 2-(9-Fluor-3-oxo-3,5,6,7-tetrahydro-2H-pyrido[1,2,3-de]-1,4-benzoxazin-8-yl)-3-thioxo-perhydroimidazo[1,5-a]pyridin-1-on | Fp.: 195°C |

**Beispiel 3.01** (Verfahren D)

8-(6,6-Dimethyl-3,5,6,7-tetrahydropyrrolo[2,1-c][1,2,4]thiadiazol-3-ylidenamino)-9-fluor-3,5,6,7-tetrahydro-2H-pyrido[1,2,3-de]-1,4-benzoxazin-3-on

Man suspendiert 0,9 g 2-Amino-4,4-dimethylpyrrolinhydrochlorid in 10 ml Methylenchlorid. Hierzu wird eine Lösung von 0,26 g Natriumhydroxid in 5 ml Wasser bei 0°C zugetropft und 30 Minuten nachgerührt. Nun wird eine Lösung von 1,5 g 9-Fluor-8-isothiocyanato-3,5,6,7-tetrahydro-2H-pyrido[1,2,3-de]-1,4-benzoxazin-3-on in 25 ml Methylenchlorid so zugetropft, daß 5°C nicht überschritten werden. Anschließend wird 3 Stunden nachgerührt, wobei die Temperatur auf 20°C ansteigt. Unter erneuter Kühlung auf -20°C wird nun eine Lösung von 0,82 g Brom in 10 ml Methylenchlorid langsam zugetropft und 1 Stunde nachgerührt, wobei die Temperatur auf 10°C ansteigt. Danach wird die Lösung mit 30 ml Wasser, 30 ml 5 %iger Natriumhydroxid-Lösung und mit 30 ml Wasser in der angegebenen Reihenfolge gewaschen. Die Methylenchloridphase wird über Magnesiumsulfat getrocknet, anschließend filtriert und eingeengt. Man nimmt den Feststoff in Essigester auf und filtriert über Kieselgel, engt anschließend erneut ein und kristallisiert aus wenig Essigester um.
Ausbeute: 0,8 g = 38 % der Theorie
Fp.: 139°C

**Beispiel 4.01** (Verfahren E)

9-Fluor-8-perhydropyrrolo[1,2-c]thiazol-3-ylidenamino)-3,5,6,7-tetrahydro-2H-pyrido[1,2,3-de]-1,4-benzoxazin-3-on

0,95 g 9-Fluor-8-(2-hydroxymethylpiperidinothiocarbonylamino)-3,5,6,7-tetrahydro-2H-pyrido[1,2,3-de]-1,4-benzoxazin-3-on werden 12 Stunden bei Raumtemperatur in 50 ml mit gasförmiger Salzsäure gesättigtem Ethanol stehen gelassen. Das Ethanol wird abdestilliert, der Rückstand in Wasser gelöst und mit Natriumhydrogencarbonat-Lösung alkalisch gestellt. Die ausgefallene Substanz wird abgesaugt und aus Isopropanol umkristallisiert.
Ausbeute: 0,53 g = 59 % der Theorie
Fp.: 203-204°C

Die folgenden Beispiele erläutern die Herstellung der neuen Zwischenprodukte zur Herstellung der erfindungsgemäßen Verbindungen:

**Beispiel 5.01**

9-Fluor-3,5,6,7-tetrahydro-2H-pyrido[1,2,3-de]-1,4-benzoxazin-3,7-dion

Man erwärmt 830 g Polyphosphorsäure auf 90°C und gibt in kleinen Portionen 103,5 g 3-(7-Fluor-3-oxo-2H-1,4-benzoxazin-4-yl)-propansäure zu. Es wird 4 Stunden bei dieser Temperatur gerührt und anschließend das Reaktionsgemisch auf 8 Liter Eiswasser gegeben. Man rührt 10 Stunden und saugt die ausgefallenen Kristalle ab. Es wird durch Mitteldruckchromatographie an Kieselgel mit Hexan/Essigestergemischen gereinigt.
Ausbeute: 59,6 g = 62 % der Theorie
Fp.: 234-235°C

In analoger Weise wurde auch die folgende Verbindung hergestellt:

| Beispiel Nr. | Name | Physikalische Konstante |
|---|---|---|
| 5.02 | 9-Fluor-5-methyl-3,5,6,7-tetrahydro-2H-pyrido[1,2,3-de]-1,4-benzoxazin-3,7-dion | Fp.: 133-134°C |

**Beispiel 6.01**

9-Fluor-7-hydroxy-3,5,6,7-tetrahydro-2H-pyrido[1,2,3-de]-1,4-benzoxazin-3-on

Man löst 8,0 g 9-Fluor-3,5,6,7-tetrahydro-2H-pyrido[1,2,3-de]-1 ,4-benzoxazin-3,7-dion in 15 ml Ethanol und gibt 0,7 g Natriumborhydrid hinzu. Man rührt 10 Stunden bei Raumtemperatur, zieht das Lösungsmittel im Vakuum ab, nimmt den Rückstand in Essigester auf, wäscht mit Wasser, trocknet über Magnesiumsulfat, filtriert und zieht das Lösungsmittel ab. Man kristallisiert aus Diethylether um.
Ausbeute: 6,4 g = 79 % der Theorie
Fp.: 166-168°C

**Beispiel 7.01**

9-Fluor-3,5-dihydro-2H-pyrido[1,2,3-de]-1,4-benzoxazin-3-on

Man löst 5,9 g 9-Fluor-7-hydroxy-3,5,6,7-tetrahydro-2H-pyrido[1,2,3-de]-1,4-benzoxazin-3-on in 300 ml Toluol und gibt 20 mg Toluolsulfonsäure hinzu. Man kocht 2 Stunden am Wasserabschneider, destilliert das Lösungsmittel ab, nimmt den Rückstand in Essigester auf und wäscht nacheinander mit Natriumhydrogencarbonat und mit Wasser. Man trocknet mit Magnesiumsulfat, filtriert und engt ein.
Ausbeute: 5,1 g = 94 % der Theorie
Fp.: 139-140°C

**Beispiel 8.01**

9-Fluor-8-nitro-3,5,6,7-tetrahydro-2H-pyrido[1,2,3-de]-1,4-benzoxazin-3-on

Man löst bei 0°C 29 g 9-Fluor-3,5,6,7-tetrahydro-2H-pyrido[1,2,3-de]-1,4-benzoxazin-3-on in konzentrierter Schwefelsäure (96 %ig) und versetzt tropfenweise bei -15°C mit einer gekühlten Lösung von 5,5 ml konzentrierter Salpetersäure (100 %ig) in 20 ml konzentrierter Schwefelsäure. Nach beendetem Zutropfen wird das Kühlbad entfernt und das Reaktionsgemisch 2 Stunden gerührt, wobei es sich bis auf 0°C erwärmt. Man gießt auf 2 Liter Eiswasser und saugt die ausgefallenen Kristalle ab. Man wäscht mit Wasser und trocknet im Vakuum.
Ausbeute: 30,7 g = 87 % der Theorie
Fp.: 166-168°C

Herstellung des Ausgangsmaterials für Beispiel 8.01

9-Fluor-3,5,6,7-tetrahydro-2H-pyrido[1,2,3-de]-1,4-benzoxazin-3-on

Unter absolutem Feuchtigkeitsausschluß werden 35 g 9-Fluor-3,5,6,7-tetrahydro-2H-pyrido[1,2,3-de]-1,4-benzoxazin-3,7-dion in 120 ml Trifluoressigsäure gelöst und anschließend 63 ml Triethylsilan zugetropft. Man erwärmt 9 Stunden auf 60°C und gießt auf 500 ml Eiswasser. Man saugt die ausgefallenen Kristalle ab und wäscht mit Eiswasser nach. Die Kristalle werden im Vakuum getocknet.

Ausbeute: 30,1 g = 92 % der Theorie
Fp.: 137-138°C

In analoger Weise wurden auch die folgenden Verbindungen hergestellt:

| Beispiel Nr. | Name | Physikalische Konstante |
| --- | --- | --- |
| 8.02 | 9-Fluor-5-methyl-8-nitro-3,5,6,7-tetrahydro-2H-pyrido[1,2,3-de]-1,4-benzoxazin-3-on | Fp.: 144-145°C |
| 8.03 | 9-Fluor-8-nitro-3,5-dihydro-2H-pyrido[1,2,3-de]-1,4-benzoxazin-3-on | Fp.: 124-125°C |

**Beispiel 9.01**

8-Amino-9-fluor-3,5,6,7-tetrahydro-2H-pyrido[1,2,3-de]-1,4-benzoxazin-3-on

Man suspendiert 29,7 g 9-Fluor-8-nitro-3,5,6,7-tetrahydro-2H-pyrido[1,2,3-de]-1 ,4-benzoxazin-3-on in 400 ml Ethanol und gibt anschließend 13,3 g Ammoniumchlorid und 200 ml Wasser zu. Man kühlt auf 0°C und gibt portionsweie 62 g Zinkpulver hinzu. Man rührt 2 Stunden bei Raumtemperatur und saugt den Feststoff ab. Man wäscht den Filterkuchen mit Essigester und engt die vereinigten Filtrate im Vakuum ein. Der Rückstand wird in Essigester aufgenommen, mit Wasser gewaschen, über Magnesiumsulfat getrocknet, filtriert und eingeengt. Der Rückstand wird aus Ether/Diisopropylether umkristallisiert.

Ausbeute: 18,0 g = 69 % der Theorie

Fp.: 112°C

In analoger Weise wurden auch die folgenden Verbindungen hergestellt:

| Beispiel Nr. | Name | Physikalische Konstante |
|---|---|---|
| 9.02 | 8-Amino-9-fluor-5-methyl-3,5,6,7-tetrahydro-2H-pyrido(1,2,3-de]-1,4-benzoxazin-3-on | zähes Öl |
| 9.03 | 8-Amino-9-fluor-3,5-dihydro-2H-pyrido-[1,2,3-de]-1,4-benzoxazin-3-on | Fp: 153-155°C |

Die folgenden Beispiele erläutern die Anwendungsmöglichkeiten der erfindungsgemäßen Verbindungen.

**Beispiel A**

Im Gewächshaus wurden die aufgeführten Pflanzenspezies nach dem Auflaufen mit den aufgeführten Verbindungen in einer Aufwandmenge von 0,3 kg Wirkstoff/ha behandelt. Die Verbindungen wurden zu diesem Zweck als Emulsionen oder Suspensionen mit 500 Litern Wasser/ha gleichmäßig über die Pflanzen versprüht. Hier zeigten zwei Wochen nach der Behandlung die erfindungsgemäßen Verbindungen eine ausgezeichnete Wirkung gegen das Unkraut. Das Vergleichsmittel zeigte nicht die gleichhohe Wirksamkeit.

In der folgenden Tabelle bedeuten:

0 = keine Schädigung
1 = 1 - 24 % Schädigung
2 = 25 - 74 % Schädigung
3 = 75 - 89 % Schädigung
4 = 90 - 100 % Schädigung
ALOMY = Alopecurus myosuroides
SETVI = Setaria viridis
SORHA = Sorghum halepense
CYPES = Cyperus esculentus
GALAP = Galium aparine

| Erfindungsgemäße Verbindung | ALOMY | SETVI | SORHA | CYPES | GALAP |
|---|---|---|---|---|---|
| Beispiel 1.01 | 3 | 3 | 3 | 3 | 4 |
| Beispiel 1.02 | 3 | 4 | 3 | 3 | 3 |
| Beispiel 1.03 | 3 | 2 | 3 | 2 | 3 |
| Beispiel 2.02 | 3 | 3 | - | 1 | 4 |
| Unbehandelt | 0 | 0 | 0 | 0 | 0 |
| Vergleichsmittel (gemäß EP-A-0 271 170) | | | | | |
| Beispiel 21 | 0 | 2 | 0 | 2 | 0 |

**Beispiel B**

Im Gewächshaus wurden die aufgeführten Pflanzenspezies vor dem Auflaufen mit den aufgeführten Verbindungen in einer Aufwandmenge von 0,3 kg Wirkstoff/ha behandelt. Die Verbindungen wurden zu diesem Zweck als Emulsionen oder Suspensionen mit 500 Litern Wasser/ha gleichmäßig über den Boden versprüht. Hier zeigten drei Wochen nach der Behandlung die erfindungsgemäßen Verbindungen eine hohe

Kulturpflanzenselektivität in Sojabohnen bei ausgezeichneter Wirkung gegen das Unkraut. Das Vergleichsmittel zeigte nicht die gleichhohe Wirksamkeit.

In der folgenden Tabelle bedeuten:

0 = keine Schädigung
1 = 1 - 24 % Schädigung
2 = 25 - 74 % Schädigung
3 = 75 - 89 % Schädigung
4 = 90 - 100 % Schädigung
GLXMA = Glycine maxima
ALOMY = Alopecurus myosuroides
AVEFA = Avena fatua
SORHA = Sorghum halepense
CYPES = Cyperus esculentus

| Erfindungsgemäße Verbindung | GLXMA | ALOMY | AVEFA | SORHA | CYPES |
|---|---|---|---|---|---|
| Beispiel 1.01 | 0 | 3 | 3 | 4 | 3 |
| Beispiel 1.02 | 0 | 4 | 2 | 3 | 3 |
| Beispiel 1.03 | 0 | 3 | 1 | 3 | 3 |
| Unbehandelt | 0 | 0 | 0 | 0 | 0 |
| Vergleichsmittel (gemäß EP-A-0 271 170) | | | | | |
| Beispiel 21 | 0 | 0 | 0 | 1 | 2 |

**Beispiel C**

Im Gewächshaus wurden die in der Tabelle aufgeführten Verbindungen mit den ebenfalls erwähnten Aufwandmengen appliziert. Hierzu wurden die Wirkstoffe in Form von Zubereitungen in Gefäße mit 1500 ml Wasser gegeben. Die Testpflanzenarten wurden im Vorauflauf sowie im 1- bis 5-Blatt-Stadium eingesetzt. Drei Wochen nach der Applikation wurde die Schädigung der Pflanzen bonitiert. Die erfindungsgemäßen Verbindungen zeigten eine starke Wirkung gegen wichtige Reisunkräuter bei gleichzeitiger Selektivität in Wasserreis. Das Vergleichsmittel zeigte nicht die gleichhohe Wirksamkeit.

In der folgenden Tabelle bedeuten:

0 = keine Schädigung
1 = schwache Schädigung
2 = mittlere Schädigung
3 = starke Schädigung
4 = total vernichtet
ORYSA = Oryza sativa
SCPJU = Scirpus juncoides

MOOVA = Monochoria vaginalis
ECHCG = Echinochloa crus-galli

| Erfindungsgemäße Verbindungen | Wasserapplikation kg Wirkstoff/ha | ORYSA | SCPJU | MOOVA | ECHCG |
|---|---|---|---|---|---|
| Beispiel 1.01 | 0,25 | 0 | 3 | 4 | - |
| Beispiel 4.01 | 0,05 | 0 | - | - | 4 |
| Vergleichsmittel | | | | | |
| Mefenacet | 0,30 | 0 | - | - | 3 |

**Beispiel D**

Im Gewächshaus wurden die aufgeführten Pflanzenspezies vor dem Auflaufen mit den aufgeführten Verbindungen in einer Aufwandmenge von 0,3 kg Wirkstoff/ha behandelt. Die Verbindungen wurden zu diesem Zweck als Emulsionen oder Suspensionen mit 500 Litern Wasser/ha gleichmäßig über den Boden versprüht. Hier zeigte drei Wochen nach der Behandlung die erfindungsgemäße Verbindung eine hohe Kulturpflanzenselektivität in Weizen und Mais bei ausgezeichneter Wirkung gegen das Unkraut. Das Vergleichsmittel zeigte nicht die gleichhohe Selektivität.

In der folgenden Tabelle bedeuten:

0 = keine Schädigung
1 = 1 - 24 % Schädigung
2 = 25 - 74 % Schädigung
3 = 75 - 89 % Schädigung
4 = 90 - 100 % Schädigung
TRZAX = Triticum aestivum
ZEAMX = Zea mays
ABUTH = Abutilon theophrasti
IPOSS = Ipomoea purpurea
MATCH = Matricaria chamomilla
SOLSS = Solanum sp.
VERPE = Veronica persica

| Erfindungsgemäße Verbindung | TRZAX | ZEAMX | ABUTH | IPOSS | MATCH | SOLSS | VERPE |
|---|---|---|---|---|---|---|---|
| Beispiel 2.01 | 0 | 0 | 4 | 4 | 4 | 4 | 4 |
| Unbehandelt | 0 | 0 | 0 | 0 | 0 | 0 | 0 |
| Vergleichsmittel | | | | | | | |
| Oxadiazon | 3 | 3 | 4 | 3 | 4 | 4 | 4 |

## Ansprüche

1. Substituierte Benzoxazolinon- und Benzoxazinon-Derivate der allgemeinen Formel I

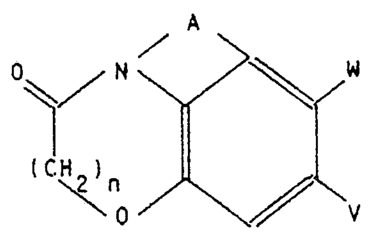

(I) ,

in der

A eine Gruppe $-CH_2-CH_2-CH_2-$, eine durch Methyl oder Ethyl subtituierte Gruppe $-CH_2-CH_2-CH_2-$, eine Gruppe $-CH=CH-CH_2-$, eine durch Methyl oder Ethyl substituierte Gruppe $-CH=CH-CH_2-$, eine Gruppe $-CH_2-CH=CH-$ oder eine durch Methyl oder Ethyl substituierte Gruppe $-CH_2-CH=CH-$,

n 0 oder 1,

V ein Wasserstoffatom, ein Fluoratom oder ein Chloratom,

W eine der Gruppen $W^1$ bis $W^4$ mit den allgemeinen Formeln

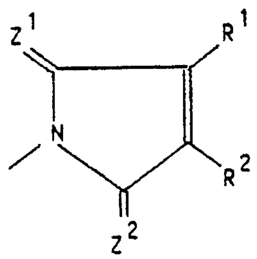

$W^1$ ,

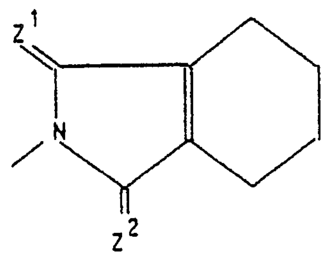

$W^2$ ,

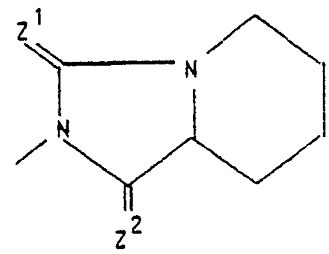

$W^3$

oder

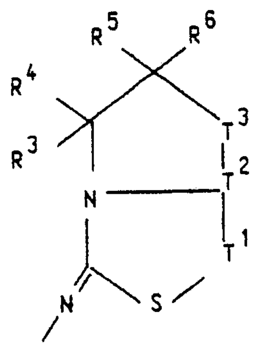

,

$W^4$

$R^1$ ein Wasserstoffatom, einen $C_1$-$C_6$-Alkylrest oder einen ein- oder mehrfach durch Halogen substituierten $C_1$-$C_6$-Alkylrest,
$R^2$ ein Wasserstoffatom, einen $C_1$-$C_6$-Alkylrest oder einen ein- oder mehrfach durch Halogen substituierten $C_1$-$C_6$-Alkylrest,
$Z^1$ ein Sauerstoffatom oder ein Schwefelatom,
$Z^2$ ein Sauerstoffatom oder ein Schwefelatom,
$T^1$-$T^2$ eine der Gruppen

$-CH_2-CH-$ oder $-N=C-$

$T^3$ die Gruppe -D-E-,
D eine Gruppe $-(CR^7R^8)_o-$,
E ein Sauerstoffatom, ein Schwefelatom oder eine der Gruppen -SO-, $-SO_2$, $-NR^9-$ oder $-CR^{10}R^{11}-$,
o 0 oder 1 und
$R^3$, $R^4$, $R^5$, $R^6$, $R^7$, $R^8$, $R^9$, $R^{10}$ und $R^{11}$ unabhängig voneinander ein Wasserstoffatom, einen $C_1$-$C_4$-Alkylrest oder einen Halogen $C_1$-$C_4$-alkylrest
bedeuten.

2. Verbindungen der allgemeinen Formel XXIII

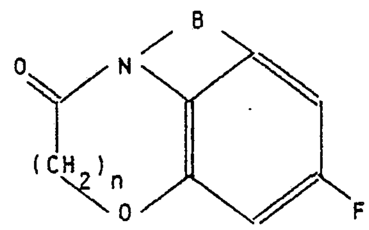

(XXIII) ,

in der
B eine durch Methyl oder Ethyl subtituierte Gruppe $-CH_2-CH_2-CH_2-$, eine Gruppe $-CH=CH-CH_2-$, eine durch Methyl oder Ethyl substituierte Gruppe $-CH=CH-CH_2-$, eine Gruppe $-CH_2-CH=CH-$, eine durch Methyl oder Ethyl substituierte Gruppe $-CH_2-CH=CH-$, eine Gruppe $-CH_2-CH_2-CHOH-$, eine durch Methyl oder Ethyl substituierte Gruppe $-CH_2-CH_2-CHOH-$, eine Gruppe $-CH_2-CH_2-CO-$oder eine durch Methyl oder Ethyl substituierte Gruppe $CH_2-CH_2-CO-$, und
n 0 oder 1
bedeuten.

3. Verbindungen der allgemeinen Formel XXIV

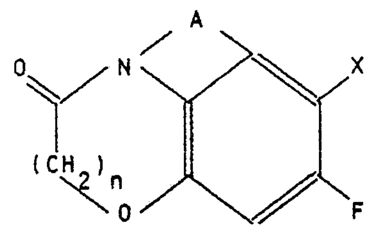

(XXIV) ,

in der
A eine Gruppe $-CH_2-CH_2-CH_2-$, eine durch Methyl oder Ethyl subtituierte Gruppe $-CH_2-CH_2-CH_2-$, eine Gruppe $-CH=CH-CH_2-$, eine durch Methyl oder Ethyl substituierte Gruppe $-CH=CH-CH_2-$, eine Gruppe $-CH_2-CH=CH-$ oder eine durch Methyl oder Ethyl substituierte Gruppe $-CH_2-CH=CH-$,
X eine Nitrogruppe oder eine Aminogruppe und
n 0 oder 1,
bedeuten.

4. Verfahren zur Herstellung von Verbindungen der allgemeinen Formel I gemäß Anspruch 1, dadurch gekennzeichnet,

A) falls W für $W^1$ oder $W^2$ und $Z^1$ und $Z^2$ für Sauerstoff stehen, eine Verbindung der allgemeinen Formel II

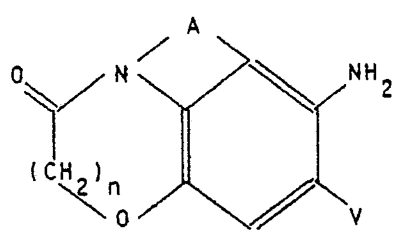

(II) ,

in der A, V und n die unter der allgemeinen Formel I genannten Bedeutungen haben, mit einer Verbindungen der allgemeinen Formeln III oder IV

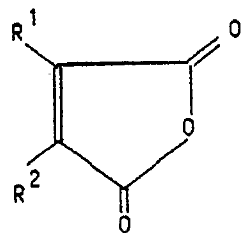

(III)

oder

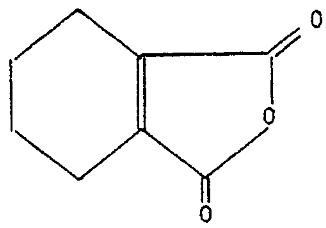

(IV) ,

in denen $R^1$ und $R^2$ die unter der allgemeinen Formel I genannten Bedeutungen haben, umsetzt,

B) falls W für $W^1$ oder $W^2$ und $Z^1$ und/oder $Z^2$ für Schwefel stehen, eine Verbindung der allgemeinen Formeln I a oder I b

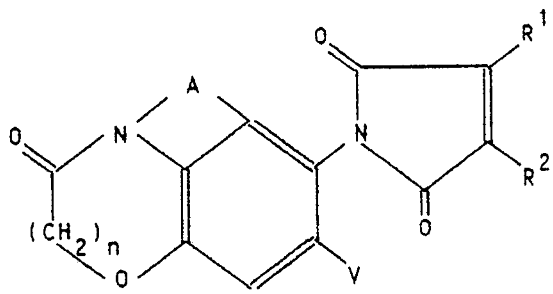

(I a)

oder

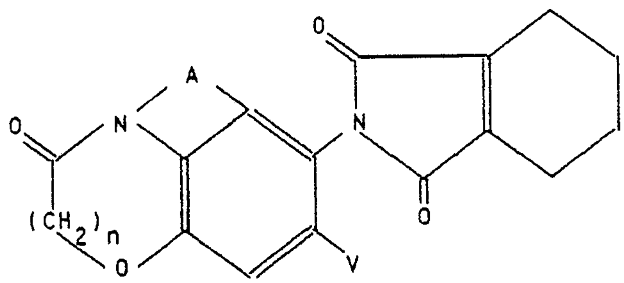

(I b) ,

in denen A, $R^1$, $R^2$, V und n die unter der allgemeinen Formel I genannten Bedeutungen haben, mit Phosphor(V)-sulfid oder Lawesson's Reagenz umsetzt,

C) falls W für $W^3$ steht, eine Verbindung der allgemeinen Formel V

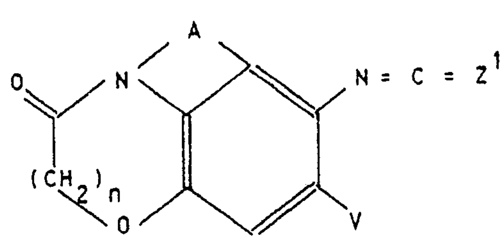

(V) ,

in der A, V, $Z^1$ und n die unter der allgemeinen Formel I genannten Bedeutungen haben, mit einer

Verbindung der allgemeinen Formel VI

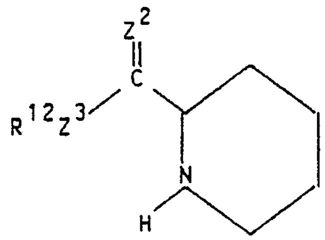

(VI) ,

in der $Z^2$ und $Z^3$ für ein Sauerstoffatom oder ein Schwefelatom und $R^{12}$ für ein Wasserstoffatom, ein Alkalimetallatom oder einen $C_1$-$C_4$-Alkylrest stehen, umsetzt,

D) falls W für $W^4$ und $T^1$-$T^2$ für die Gruppe

$$-N=C-$$

- steht, eine Verbindung der allgemeinen Formel Va

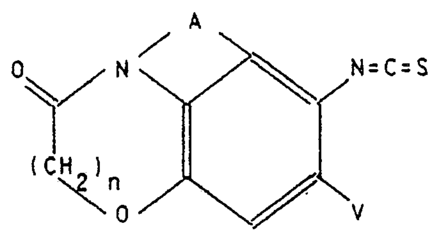

(V a),

in der A, V und n die unter der allgemeinen Formel I genannten Bedeutungen haben, mit einer Verbindung der allgemeinen Formel VII

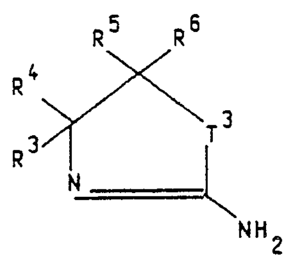

(VII),

in der $R^3$, $R^4$, $R^5$, $R^6$ und $T^3$ die unter der allgemeinen Formel I genannten Bedeutungen haben, umsetzt und in Gegenwart eines Oxidationsmittels zum Thiadiazolring cyclisiert,

E) falls W für $W^4$ and $T^1$-$T^2$ für die Gruppe

$$-CH_2-CH-$$

steht, eine Verbindung der allgemeinen Formel VIII

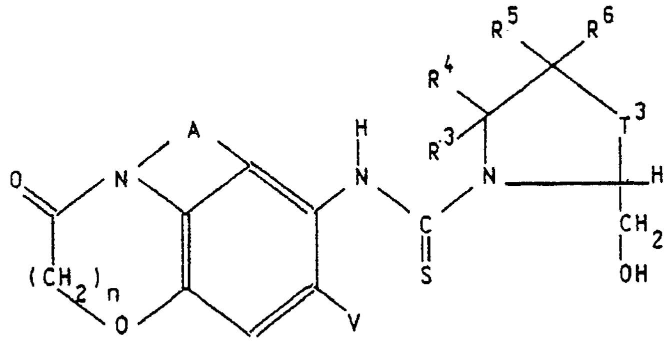

(VIII) ,

in der A, $R^3$, $R^4$, $R^5$, $R^6$, $T^3$, V und n die unter der allgemeinen Formel I genannten Bedeutungen haben, unter sauren Bedingungen cyclisiert.

5. Mittel mit herbizider Wirkung, gekennzeichnet durch einen Gehalt an mindestens einer Verbindung gemäß dem Anspruch 1.

6. Verwendung von Mitteln gemäß dem Anspruch 5 zur Bekämpfung monokotyler und dikotyler Unkrautarten in landwirtschaftlichen Hauptkulturen.

7. Verfahren zur Herstellung von Mitteln mit herbizider Wirkung, dadurch gekennzeichnet, daß man Verbindungen der allgemeinen Formel I gemäß dem Anspruch 1 mit Träger- und/oder Hilfsstoffen vermischt.